# EUROPÄISCHE PATENTSCHRIFT

(11) **EP 1 351 909 B1**
(45) Veröffentlichungstag und Bekanntmachung des Hinweises auf die Patenterteilung: **05.04.2006**
(21) Anmeldenummer: 02708262.7
(22) Anmeldetag: 08.01.2002
(51) Int. Cl.: C07C 29/80, C07C 31/20, B01D 3/14

(54) **VERFAHREN ZUR DESTILLATIVEN AUFARBEITUNG VON 1,6-HEXANDIOL, 1,5-PENTANDIOL UND CAPROLACTON**
METHOD FOR THE DISTILLATIVE PROCESSING OF 1,6-HEXANDIOL, 1,5-PENTANDIOL AND CAPROLACTONE
PROCEDE DE TRAITEMENT PAR DISTILLATION DE 1,6-HEXANDIOL, DE 1,5-PENTANDIOL ET DE CAPROLACTONE

(30) Priorität: 09.01.2001 DE 10100552
(43) Veröffentlichungstag der Anmeldung: 15.10.2003
(73) Patentinhaber: BASF Aktiengesellschaft, 67056 Ludwigshafen (DE)
(72) Erfinder: GALL, Martin, 67112 Mutterstadt (DE); KAIBEL, Gerd, 68623 Lampertheim (DE); KRUG, Thomas, 67549 Worms (DE); RUST, Harald, 67435 Neustadt (DE); STEIN, Frank, 67098 Bad Dürkheim (DE)
(74) Vertreter: Isenbruck, Günter
(86) Internationale Anmeldenummer: PCT/EP2002/000105
(87) Internationale Veröffentlichungsnummer: WO 2002/055460

(56) Entgegenhaltungen:
- EP-A- 0 122 367
- EP-A- 0 380 001
- EP-A- 0 804 951
- DE-A- 19 607 954
- DE-A- 19 618 152

## Beschreibung

Die Erfindung betrifft ein Verfahren zur destillativen Aufarbeitung der im Verfahren gemäß DE-A 196 07 954 erhaltenen Rohprodukte 1,6-Hexandiol, 1,5-Pentandiol und Caprolacton, im folgenden abgekürzt als HDO, PDO bzw. CLO bezeichnet, sowie die Verwendung eine Trennkolonne oder thermisch gekoppelten Kolonnen zur Durchführung des Verfahrens.

HDO, PDO und CLO sind wichtige Monomerbausteine, insbesondere für die Polyester-und Polyuretanherstellung. Die genannten Substanzen können, mit der für den aufgeführten Einsatzzweck erforderlichen hohen Reinheit, bevorzugt von mindestens 99%, insbesondere von 1,4-Cyclohexandiolen praktisch frei, nach dem in der nicht vorveröffentlichten DE-A 196 07 954, die hiermit voll umfänglich in den Offenbarungsgehalt der vorliegenden Erfindung einbezogen wird, beschriebenen Verfahren aus einem komplexen Carbonsäuregemisch erhalten werden, das als Nebenprodukt der Oxidation von Cyclohexan zu Cyclohexanon/Cyclohexanol anfällt. Hierbei ist bereits das Ausgangsgemisch, in der Regel als Dicarbonsäurelösung (DCL) bezeichnet, ein komplexes Gemisch aus einer Vielzahl von Substanzen. Daraus wird, nach dem in der genannten Anmeldung beschriebenen mehrstufigen Verfahren in Stufe 5 ein Hydrieraustrag erhalten, aus dem in Stufe 6 destillativ ein überwiegend 1,6-Hexandiol neben 1,5-Pentandiol enthaltender Strom erhalten wird. Daraus wird in Stufe 7 durch destillative Auftrennung ein 1,5-Pentandiol enthaltender Kopfstrom, woraus destillativ 1,5-Pentandiol als Reinprodukt gewonnen wird, sowie ein 1,6-Hexandiol als Reinprodukt enthaltender Seitenstrom entnommen.

Durch Cyclisierung eines überwiegend 6-Hydroxycapronsäureester enthaltenden Stroms in Stufe 13 wird Caprolacton gewonnen, das in Stufe 14 destillativ aufgearbeitet wird. Aufgrund der komplexen Stoffgemische war es bereits überraschend, daß trotz der ungünstigen Siedepunktverhältnisse und zu befürchtenden Azeotropbildung die Zielprodukte HDO, PDO und CLO destillativ mit hoher Reinheit, insbesondere HDO mit einem sehr geringen verbleibenden 1,4-Cyclohexandiolgehalt erhalten werden konnten.

Zur destillativen Auftrennung von Mehrkomponentengemischen sind sogenannte Trennwandkolonnen bekannt, das heißt Destillationskolonnen mit senkrechten Trennwänden, die in Teilbereichen eine Quervermischung von Flüssigkeits- und Brüdenströmen verhindern. Die Trennwand, die vorzugsweise aus einem Blech bestehen kann, unterteilt die Kolonne in Längsrichtung in deren mittleren Bereich in einen Zulaufteil und einen Entnahmeteil.

Ein ähnliches Ergebnis kann mit sogenannten thermisch gekoppelten Kolonnen erreicht werden, das heißt Anordnungen von mindestens zwei Kolonnen, wobei jede der Kolonnen mit jeder anderen mindestens zwei Verknüpfungen an räumlich getrennten Stellen aufweist.

Aufgabe der Erfindung war es, ein verbessertes, insbesondere wirtschaftlicheres Verfahren zur Gewinnung der Reinprodukte HDO, PDO und CLO aus den im Verfahren gemäß der DE-A 196 07 954 anfallenden entsprechenden Rohprodukte zur Verfügung zu stellen.

Die Lösung geht aus von einem Verfahren zur destillativen Aufarbeitung der im Verfahren gemäß DE-A 196 07 954 erhaltenen 1,6-Hexandiol (HDO), 1,5-Pentandiol (PDO) bzw. Caprolacton (CLO) enthaltenden Rohprodukte zur Gewinnung der entsprechenden Reinprodukte.

Die Erfindung ist dadurch gekennzeichnet, daß die destillative Aufarbeitung jeweils in einer Trennwandkolonne (TK), in der eine Trennwand (T) in Kolonnenlängsrichtung unter Ausbildung eines oberen gemeinsamen Kolonnenbereichs eines unteren gemeinsamen Kolonnenbereichs, eines Zulaufteils mit Verstärkungsteil und Abtriebsteil sowie eines Entnahmeteils mit Abtriebsteil und Verstärkungsteil angeordnet ist, mit Zuführung des jeweiligen Rohprodukts HDO, PDO oder CLO im Bereich des Zulaufteils, Abführung der Hochsiederfraktion (C) aus dem Kolonnensumpf, der Leichtsiederfraktion (A) über den Kolonnenkopf und der Mittelsiederfraktion (B) aus dem Bereich des Entnahmeteils oder in thermisch gekoppelten Kolonnen durchgeführt wird.

Es wurde überraschend gefunden, daß die anspruchsvolle destillative Trennaufgabe zur Gewinnung der Reinprodukte HDO, PDO und CLO aus den entsprechenden, im Verfahren gemäß DE-A 196 07 954 anfallenden Rohprodukten auch in den bekanntermaßen schwieriger zu beherrschenden Trennwandkolonnen bzw. thermisch gekoppelten Kolonnen erfolgreich gelöst werden kann.

Die genannten Rohprodukte sind komplexe Gemische, die typischerweise Zusammensetzungen wie nachstehend aufgeführt aufweisen; hierbei werden, wie üblich, als Leichtsieder Substanzen bezeichnet, deren Siedepunkt unterhalb des jeweiligen Hauptprodukts und als Hochsieder Substanzen, deren Siedepunkt oberhalb des jeweiligen Hauptprodukts liegt:
Roh-HDO enthält neben dem Hauptprodukt HDO in der Regel ca. 15 bis 23 Gew.% Leichtsieder, hiervon insbesondere PDO, 1,2-Cyclohexandiol, Hexanol, Butandiol und Caprolacton, daneben ca. 2 bis 4% Hochsieder, insbesondere Di-HDO-Ether und Hydroxycapronsäure-HDO-Ether.
Roh-PDO enthält neben dem Hauptprodukt PDO in der Regel ca. 15 bis 30 Gew.-% Leichtsieder (1,2-Cyclohexandiol, Hexanol, Butandiol), daneben ca. 20 bis 50 Gew.% Hochsieder, insbesondere HDO.
Roh-CLO enthält neben dem Hauptprodukt CLO in der Regel 1,5 bis 3,0 Gew.-% Leichtsieder, vorzugsweise Methanol, Valerolacton, ungesättigtes Valerolacton, Ameisensäure-PDO-Ester sowie ca. 0,1 bis 1 Gew.-% Hochsieder, insbesondere dimeres CLO, Ameisensäure-Hydroxycapronsäure-Methylester und Hydroxycapronsäure-Methylester.

Unter dem Begriff Reinprodukt wird vorliegend, bezogen auf HDO, PDO bzw. CLO, jeweils ein Gemisch verstanden, das wie nachstehend definiert ist:
Rein-HDO enthält zumindest 98 Gew.-%, insbesondere zumindest 99 Gew.-%, besonders bevorzugt zumindest 99,7 Gew.-% 1,6-Hexandiol, Rest Verunreinigungen, insbesondere Heptandiol, 1,4-Cyclohexandiol, 1,2-Cyclohexandiol und PDO.
Rein-PDO enthält mindestens 93 Gew.-%, insbesondere mindestens 95 Gew.-%, besonders bevorzugt mindestens 97 Gew.-% 1,5-Pentandiol, Rest Verunreinigungen, hauptsächlich HDO, 1,4-Cyclohexandiol, CLO, 1,2-Cyclohexandiol sowie 1,4-Butandiol.
Rein-CLO enthält mindestens 99 Gew.%, insbesondere mindestens 99,5 Gew.-%, besonders bevorzugt mindestens 99,9 Gew.-% Caprolacton, Rest Verunreinigungen, hauptsächlich Hydroxycapronsäure-Methylester, Ameisensäure-Hydroxycapronsäure-Methylester, 1,2-Cyclohexandiol, Ameisensäure, PDO-Ester sowie Valerolacton.

Trennwandkolonnen weisen typischerweise eine in Kolonnenlängsrichtung ausgerichtete Trennwand auf, die den Kolonneninnenraum in die folgenden Teilbereiche unterteilt: einen oberen gemeinsamen Kolonnenbereich einen unteren gemeinsamen Kolonnenbereich sowie einen Zulaufteil und einen Entnahmeteil, jeweils mit Verstärkungsteil und Abtriebsteil. Das aufzutrennende Gemisch wird im Bereich des Zulaufteils aufgegeben, eine Hochsiederfraktion wird aus dem Kolonnensumpf, eine Leichtsiederfraktion über den Kolonnenkopf und eine Mittelsiederfraktion aus dem Bereich des Entnahmeteils entnommen.

Bei der Trennung von Mehrstoffgemischen in eine Leichtsieder-, eine Mittelsieder- und eine Hochsiederfraktion werden üblicherweise Spezifikationen über den maximal zulässigen Anteil an Leichtsiedern und Hochsiedem in der Mittelsiederfraktion vorgegeben. Hierbei werden für das Trennproblem kritische Komponenten, sogenannte Schlüsselkomponenten, spezifiziert. Dabei kann es sich um eine einzelne Schlüsselkomponente oder um die Summe von mehreren Schlüsselkomponenten handeln. Im vorliegenden Verfahren sind Schlüsselkomponenten der HDO-Reindestillation BDO (Leichtsieder) und Heptandiol (Hochsieder). Schlüsselkomponenten der PDO-Reindestillation sind 1,2-Cyclohexandiol (Leichtsieder) und HDO (Hochsieder). Schlüsselkomponenten der CLO-Reindestillation sind Valerolacton (Leichtsieder) und Hydroxycapronsäure-Methylester (Hochsieder).

In einer bevorzugten Verfahrensvariante wird die Einhaltung der Spezifikation bezüglich der Schlüsselkomponenten gewährleistet, indem das Aufteilungsverhältnis der Flüssigkeit am oberen Ende der Trennwand sowie die Heizleistung der Verdampfer in bestimmter Weise geregelt werden. Dabei wird das Aufteilungsverhältnis der Flüssigkeit am oberen Ende der Trennwand in der Weise eingestellt, daß der Anteil an hochsiedenden Schlüsselkomponenten im Flüssigkeitsrücklauf über den Abtriebsteil des Entnahmeteils 10 bis 80%, bevorzugt 30 bis 50% des in der Mittelsiederfraktion zugelassenen Grenzwerts beträgt, und die Heizleistung im Sumpfverdampfer der Trennwandkolonne in der Weise eingestellt wird, daß die Konzentration der leichtsiedenden Schlüsselkomponenten in der Flüssigkeit am unteren Ende der Trennwand 10 bis 80, bevorzugt 30 bis 50% des im Mittelsiederstrom zugelassenen Grenzwerts , beträgt. Entsprechend wird bei dieser Regelung die Flüssigkeitsaufteilung am oberen Ende der Trennwand dahingehend eingestellt, daß bei höheren Gehalten an hochsiedenden Schlüsselkomponenten mehr und bei geringeren Gehalten derselben weniger Flüssigkeit auf den Zulaufteil geleitet wird. Analog wird die Regelung der Heizleistung dahingehend vorgenommen, daß bei höherem Gehalt an leichtsiedenden Schlüsselkomponenten die Heizleistung erhöht und bei niedrigerem Gehalt derselben die Heizleistung verringert wird.

Es wurde gefunden, daß eine weitere Verbesserung des Verfahrens erreicht werden kann, indem durch entsprechende Regelvorschriften eine weitgehend gleichmäßige Beaufschlagung mit Flüssigkeit gesichert wird. Störungen der Zulaufmenge oder der Zulaufkonzentration werden kompensiert. Hierzu wird erfindungsgemäß sichergestellt, daß der Mengenstrom der Flüssigkeit, die den unteren Teil des Zulaufteils beaufschlagt, nicht unter 30% seines Normalwerts sinkt.

Bevorzugt wird auch die Aufteilung der aus dem Abtriebsteil des. Entnahmeteils der Trennwandkolonne ablaufenden Flüssigkeit auf die abgezogene Mittelsiederfraktion und den Verstärkungsteil des Entnahmeteils der Trennwandkolonne in der Weise geregelt, daß die auf den Verstärkungsteil aufgegebene Flüssigkeitsmenge nicht unter 30% ihres Normalwerts sinkt.

Bevorzugt werden die HDO-Reinkolonnen und die PDO-Reinkolonnen so verschaltet, daß die im Kopfstrom der HDO-Kolonne verbleibenden HDO-Anteile über den Sumpf der PDO-Reinkolonne gewonnen und in die HDO-Reinkolonne zurückgeführt werden.

Die Mittelsiederfraktion wird bevorzugt in flüssiger Form entnommen; diese Verfahrensvariante ist thermisch vorteilhaft und apparativ einfacher zu realisieren.

In einer bevorzugten Verfahrensvariante kann der Brüdenstrom am unteren Ende der Trennwand so eingestellt werden, daß das Mengenverhältnis des Brüdenstroms im Zulaufteil zum Brüdenstrom im Entnahmeteil 0,8 bis 1,2, bevorzugt 0,9 bis 1,1 beträgt, vorzugsweise durch die Wahl und/oder Dimensionierung trennwirksamer Einbauten und/oder den Einbau druckverlusterzeugender Einrichtungen.

In einer weiteren bevorzugten Verfahrensvariante kann der Rücklauf aus dem oberen gemeinsamen Kolonnenteil so geregelt werden, daß das Verhältnis des Rücklaufstroms im Zulaufteil zum Rücklauf im Entnahmeteil 0,1 bis 1, bevorzugt 0,5 bis 0,8 beträgt.

Weiter bevorzugt kann die Entnahme des Kopfstroms temperaturgeregelt erfolgen, wobei die Meßstelle für die Regeltemperatur im oberen gemeinsamen Teilbereich der Kolonne, an einer Stelle angeordnet ist, die um 3 bis 8, bevorzugt um 4 bis 6 theoretische Trennstufen unterhalb des oberen Kolonnenendes angeordnet ist.

Entsprechend einer weiteren bevorzugten Verfahrensvariante kann die Entnahme des Hochsiederstroms temperaturgeregelt erfolgen, wobei die Meßstelle für die Regeltemperatur im unteren gemeinsamen Kolonnenbereich um 3 bis 8, bevorzugt um 4 bis 6 theoretische Trennstufen oberhalb des unteren Endes der Kolonne angeordnet ist.

Gemäß einer weiteren Verfahrensvariante erfolgt die Entnahme des Mittelsiederstroms standgeregelt, wobei als Regelgröße der Flüssigkeitsstand im Verdampfer oder im Sumpf der Kolonne verwendet wird.

Gegenstand der Erfindung ist auch die Verwendung einer Trennwandkolonne zur Durchführung des erfindungsgemäßen Verfahrens. Besonders geeignet sind hierzu Trennwandkolonnen mit 30 bis 100, bevorzugt mit 50 bis 90 theoretischen Trennstufen.

Die Aufteilung der Trennstufenzahl auf die einzelnen Teilbereiche der Trennwandkolonne erfolgt dabei bevorzugt in der Weise, daß jeder der 6 Kolonnenbereiche der Trennwandkolonne jeweils 5 bis 50%, bevorzugt 15 bis 30% der Gesamtzahl der theoretischen Trennstufen der Trennwandkolonne aufweist.

In einer bevorzugten Ausgestaltung der Trennwandkolonne können die Zulaufstelle des aufzutrennenden Stroms und die Entnahmestelle des Mittelsiederstroms auf unterschiedlichen Höhe in der Kolonne angeordnet sein, vorzugsweise um 1 bis 20, insbesondere um 10 bis 15 theoretische Trennstufen beabstandet.

Bezüglich der einsetzbaren trennwirksamen Einbauten in der Trennwandkolonne gibt es grundsätzlich keine Einschränkungen: hierzu sind sowohl Füllkörper als auch geordnete Packungen oder Böden geeignet. Aus Kostengründen werden bei Kolonnen mit einem Durchmesser über 1,2 m in der Regel Böden, bevorzugt Ventil- oder Siebböden eingesetzt.

Bei den Packungskolonnen sind geordnete Blechpackungen mit einer spezifischen Oberfläche von 100 bis 500 m²/m³, bevorzugt von etwa 250 bis 300 m²/m³ besonders geeignet.

In einer bevorzugten Verfahrensvariante ist die Flüssigkeitsverteilung in den einzelnen Teilbereichen der Trennwandkolonne jeweils getrennt einstellbar. Dadurch kann der Gesamtenergiebedarf, der zur Auftrennung des Gemisches benötigt wird, minimiert werden.

Besonders vorteilhaft kann in den Teilbereichen des Zulaufteils der Trennwandkolonne die Flüssigkeit verstärkt im Wandbereich und in Teilbereichen der Trennwandkolonne reduziert im Wandbereich aufgegeben werden. Hierdurch werden unerwünschte Schleichströme vermieden und die erzielbaren Produkt-Endreinheiten gesteigert.

Die Trennwandkolonne kann in einem oder mehreren Teilbereichen mit geordneten Packungen oder Füllkörpern bestückt sein.

Es ist möglich, die Trennwand in Form von lose gesteckten Teilsegmenten auszugestalten. Dies führt zur weiteren Kostensenkung bei der Herstellung und Montage der Trennwandkolonne.

Besonders vorteilhaft kann die lose Trennwand interne Mannlöcher oder herausnehmbare Segmente aufweisen, die es erlauben, innerhalb der Trennwandkolonne von einer Seite der Trennwand auf die andere Seite zu gelangen.

Bei besonders hohen Anforderungen an die Produktreinheit ist es günstig, insbesondere für den Fall, daß Packungen als trennwirksame Einbauten eingesetzt werden, die Trennwand mit einer thermischen Isolierung auszustatten. Eine derartige Ausgestaltung der Trennwand ist beispielsweise in EP-A-0 640 367 beschrieben. Besonders günstig ist eine doppelwandige Ausführung mit dazwischenliegendem engem Gasraum.

Erfindungsgemäß ist es auch möglich, anstelle der Trennwandkolonne thermisch gekoppelte Kolonnen einzusetzen. Anordnungen mit thermisch gekoppelten Kolonnen sind hinsichtlich des Energiebedarfs mit einer Trennwandkolonne gleichwertig. Diese Erfindungsvariante bietet sich insbesondere bei Verfügbarkeit von bestehenden Kolonnen an. Die geeignetsten Formen der Zusammenschaltung können je nach Trennstufenzahl der vorhandenen Kolonnen ausgewählt werden.

Die thermisch gekoppelten Kolonnen können somit jeweils mit einem eigenen Verdampfer und/oder Kondensator ausgestattet sein.

In einer bevorzugten Verfahrensvariante werden in den Verbindungsströmen zwischen den beiden thermisch gekoppelten Kolonnen nur Flüssigkeiten gefördert. Dies ist besonders vorteilhaft, wenn die thermisch gekoppelten Kolonnen mit unterschiedlichen Drücken betrieben werden.

In einer bevorzugten Verschaltung der thermisch gekoppelten Kolonne werden die Leichtsiederfraktion und die Hochsiederftaktion aus unterschiedlichen Kolonnen entnommen, wobei der Betriebsdruck der Kolonne, aus der die Hochsiederfraktion entnommen wird, tiefer eingestellt wird als der Betriebsdruck der Kolonne, aus der die Leichtsiederfraktion entnommen wird, bevorzugt um 0,1 bis 2 bar, insbesondere um 0,5 bis 1 bar.

Gemäß einer besonderen Verschaltungsfonn ist es möglich, den Sumpfstrom der ersten Kolonne in einem Verdampfer teilweise oder vollständig zu verdampfen und anschließend der zweiten Kolonne zweiphasig oder in Form eines gasförmigen und eines flüssigen Stromes zuzuführen.

Das erfindungsgemäße Verfahren kann bevorzugt sowohl bei der Verwendung einer Trennwandkolonne als auch von thermisch gekoppelten Kolonnen in der Weise geführt werden, daß der Zulaufstrom teilweise oder vollständig vorverdampft wird und der Kolonne zweiphasig oder in Form eines gasförmigen und eines flüssigen Stromes zugeführt wird.

Diese Vorverdampfung bietet sich insbesondere dann an, wenn der Sumpfstrom der ersten Kolonne größere Mengen an Mittelsiedern enthält. In diesem Fall kann die Vorverdampfung auf einem niedrigeren Temperaturniveau erfolgen und der Verdampfer der zweiten Kolonne entlastet werden. Weiterhin wird durch diese Maßnahme der Abtriebsteil der zweiten Kolonne wesentlich entlastet. Der vörverdampfte Strom kann dabei der zweiten Kolonne zweiphasig oder in Form von zwei separaten Strömen zugeführt werden.

Die Trennwandkolonne zur Durchführung des erfindungsgemäßen Verfahrens weist am oberen und am unteren Ende der Trennwand Probenahmemöglichkeiten auf, über die aus der Kolonne kontinuierlich oder in zeitlichen Abständen flüssige und/oder gasförmige Proben entnommen und hinsichtlich ihrer Zusammensetzung, bevorzugt gaschromatographisch, untersucht werden.

In der Ausführungsvariante mit thermisch gekoppelten Kolonnen sind die Probenahmemöglichkeiten analog, in den Verbindungsleitungen zwischen den den Teilbereichen der Tennwandkolonne entsprechenden Bereichen der thermisch gekoppelten Kolonnen angeordnet.

Die Erfindung wird im folgenden anhand einer Zeichnung sowie von Ausführungsbeispielen näher erläutert.

Es zeigt:

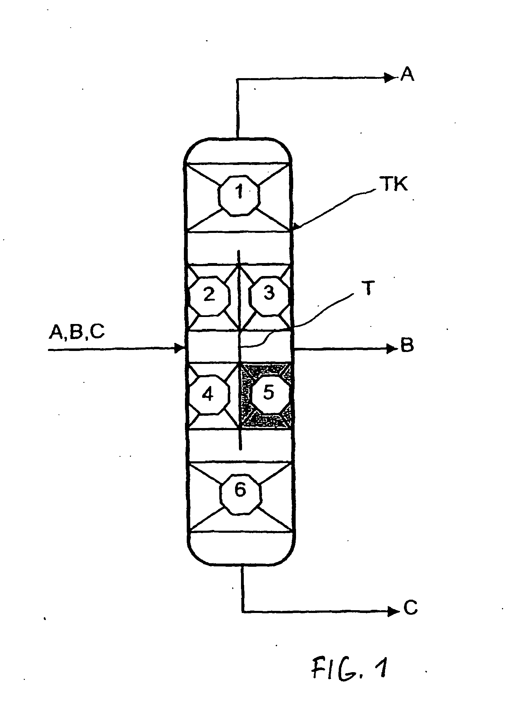

- Fig.1: die schematische Darstellung einer Trennwandkolonne zur Durchführung des erfindungsgemäßen Verfahrens.

Figur 1 zeigt schematisch eine Trennwandkolonne (TK) mit darin vertikal angeordneter Trennwand (T), die die Kolonne in einen oberen gemeinsamen Kolonnenbereich 1, einen unteren gemeinsamen Kolonnenbereich 6, einen Zulaufteil 2, 4 mit Verstärkungsteil 2 und Abtriebsteil 4 sowie einen Entnahmeteil 3, 5 mit Abtriebsteil 3 und Verstärkurigsteil 5 aufteilt. Die Zuführung des aufzutrennenden Gemisches (A, B, C) erfolgt im mittleren Bereich des Zulaufteils 2, 4. Am Kolonnenkopf wird die Leichtsiederfraktion (A), aus dem Kolonnensumpf die Hochsiederfraktion (C) und aus dem mittleren Bereich des Entnahmeteils 3, 5 die Mittelsiederfraktion (B) abgezogen.

Einer erfindungsgemäßen Trennwandkolonne, die mit Drahtgewebefüllkörpern bestückt war, 85 theoretische Trennstufen aufwies, hiervon 18 Trennstufen im unteren gemeinsamen Kolonnenbereich 6, 47 Trennstufen im Bereich der Trennwand sowie 20 Trennstufen im oberen gemeinsamen Kolonnenbereich 1 wurde ein Roh-HDO-Strom zugeführt, der neben HDO als Hauptkomponente ca. 20 Gew.-% Leichtsieder, hiervon überwiegend PDO (ca. 10 Gew.-%), 1,2-Cyclohexandiol (ca. 4 Gew.-%) und Hexanol (ca. 0,2 Gew.-%), ca. 2,5 Gew.-% Hochsieder, hiervon als Hauptkomponente Di-HDO-Ether, in einem Anteil von 2 Gew.-% enthielt. Sowohl in der Leichtsieder- wie auch in der Hochsiederfraktion waren darüber hinaus eine Vielzahl weiterer Komponenten in jeweils geringer Konzentration enthalten.

### Beispiel 1

Die Trennwandkolonne wurde mit einem Kopfdruck von 150 mbar und einem Rückflußverhältnis von 20 betrieben. Die Flüssigkeit am oberen Ende der Trennwand wurde zu gleichen Teilen auf den Zulaufteil und auf den Entnahmeteil der Trennwandkolonne aufgeteilt. Der Roh-HDO-Strom wurde auf der 52. theoretischen Trennstufe aufgegeben, das Produkt Rein-HDO wurde von der 28. theoretischen Trennstufe abgezogen. Es wurde ein spezifikationsgerechtes Produkt erhalten, d.h. ein Rein-HDO mit einem Gewichtsanteil an HDO von 99 Gew.-%.

### Beispiel 2

Für die destillative Gewinnung von Rein-PDO aus Roh-PDO wurde die selbe Apparatur wie zu HDO (Beispiel 1) eingesetzt. Die Trennwandkolonne wurde jedoch mit einem Kopfdruck von 100 mbar und einem Rücklaufverhältnis von 40 betrieben. Die Flüssigkeit am Kopf der Trennwand wurde zu ca. 44% auf den Zulaufteil und zu den verbliebenen 56% auf den Entnahmeteil aufgeteilt. Der Zulaufstrom, das Roh-PDO enthielt neben PDO als Hauptkomponente 25 Gew.-% Leichtsieder, hiervon ca. 13 Gew.-% 1,2-Cyclohexandiol, ca. 1,5 Gew.-% Butandiol sowie ca. 2,6 Gew.-% Valerolacton und ca. 45 Gew.-% Hochsieder, hiervon als Hauptkomponente ca. 43 Gew.-% HDO.

Das Roh-PDO wurde auf die Trennwandkolonne auf der 48. theoretischen Trennstufe aufgegeben, das Produkt Rein-PDO wurde von der 40. theoretischen Trennstufe abgezogen. Es wurde ein spezifikationsgerechtes Rein-PDO erhalten, d.h. ein Produkt mit einem Anteil an PDO von mindestens 97 Gew.-%.

### Beispiel 3

In der selben Kolonne wie zu Beispiel 1 beschrieben, wurde ein Roh-CLO aufgegeben, das neben CLO als Hauptkomponente ca. 2,5 Gew.% Leichtsieder, hiervon überwiegend Methanol (0,9 Gew.-%) und Valerolacton (0,4 Gew.-%) sowie ca. 0,5 Gew.-% Hochsieder, hiervon überwiegend dimeres Caprolacton (ca. 0,05 Gew.-%), Ameisensäure-Hdroxycapronsäure-Methylester (ca. 0,02 Gew.-%) sowie Hydroxycapronsäure-Methylester (ca. 0,02 Gew.-%), enthielt. In der Leichtsieder- wie auch in der Hochsiederfraktion waren darüber hinaus eine Vielzahl von Komponenten in jeweils geringerer Konzentration enthalten.

Die Trennwandkolonne wurde mit einem Kopfdruck von 50 mbar und einem Rückflußverhältnis von 38 betrieben. Die Flüssigkeit am oberen Ende der Trennwand wurde zu ca. 33% auf den Zulaufteil und zu ca. 66% auf den Entnahmeteil aufgeteilt. Das Roh-PDO wurde auf der 32. theoretischen Trennstufe aufgegeben, das Produkt Rein-CLO wurde von der 32. theoretischen Trennstufe entnommen. Es wurde ein spezifikationsgerechtes Rein-CLO erhalten, d.h. ein Produkt, das mindestens 99 Gew.-% CLO enthält.

## Patentansprüche

1. Verfahren zur destillativen Aufarbeitung der im Verfahren gemäß DE-A 196 07 954 erhaltenen 1,6-Hexandiol (HDO), 1,5-Pentandiol (PDO) bzw. Caprolacton (CLO) enthaltenden Rohprodukte zur Gewinnung der entsprechenden Reinprodukte, **dadurch gekennzeichnet, daß** die destillative Aufarbeitung jeweils in einer Trennwandkolonne (TK), in der eine Trennwand (T) in Kolonnenlängsrichtung unter Ausbildung eines oberen gemeinsamen Kolonnenbereichs (1), eines unteren gemeinsamen Kolonnenbereichs (6), eines Zulaufteils (2, 4) mit Verstärkungsteil (2) und Abtriebsteil (4) sowie eines Entnahmeteils (3, 5) mit Abtriebsteil (3) und Verstärkungsteil (5) angeordnet ist, mit Zuführung des jeweiligen Rohprodukts HDO, PDO bzw. CLO im mittleren Bereich des Zulaufteils (2, 4), Abführung einer Hochsiederfraktion (C) aus dem Kolonnensumpf, einer Leichtsiederfraktion (A) über den Kolonnenkopf und einer Mittelsiederfraktion (B) aus dem mittleren Bereich des Entnahmeteils (3, 5) oder in thermisch gekoppelten Kolonnen durchgeführt wird.

2. Verfahren nach Anspruch 1, **dadurch gekennzeichnet, daß** das Aufteilungsverhältnis des Flüssigkeitsrücklaufs am oberen Ende der Trennwand (T) in der Weise eingestellt wird, daß der Anteil an hochsiedenden Schlüsselkomponenten im Flüssigkeitsrücklauf über den Abtriebsteil (3) des Entnahmeteils (3, 5) am oberen Ende der Trennwand (T) 10 bis 80 %, bevorzugt 30 bis 50 % des in der Mittelsiederfraktion (B) zugelassenen Grenzwerts beträgt, und daß die Heizleistung im Sumpfverdampfer der Trennwandkolonne (TK) in der Weise eingestellt wird, daß die Konzentration der leichtsiedenden Schlüsselkomponenten in der Flüssigkeit am unteren Ende der Trennwand (T) 10 bis 80 %, bevorzugt 30 bis 50 % des im Mittelsiederstrom (B) zugelassenen Grenzwerts beträgt.

3. Verfahren nach Anspruch 1 oder 2, **dadurch gekennzeichnet, daß** die HDO-Reinkolonne und die PDO-Reinkolonne so verschaltet werden, daß die im Kopfstrom der HDO-Kolonne verbleibenden HDO-Anteile über den Sumpf der PDO-Reinkolonne gewonnen und in die HDO-Reinkolonne zurückgeführt werden.

4. Verfahren nach einem der Ansprüche 1 bis 3, **dadurch gekennzeichnet, daß** der Mengenstrom der Flüssigkeit, die im mittleren Bereich des Zulaufteils (2, 4) aufgegeben wird, in der Weise geregelt wird, daß er nicht unter 30 % seines Normalwerts sinkt.

5. Verfahren nach einem der Ansprüche 1 bis 4, **dadurch gekennzeichnet, daß** die Aufteilung der aus dem Abtriebsteil (3) des Entnahmeteils (3, 5) der Trennwandkolonne (TK) ablaufenden Flüssigkeit auf die abgezogene Mittelsiederfraktion (B) und den Verstärkungsteil (5) des Entnahmeteils (3, 5) der Trennwandkolonne (TK) durch eine Regelung in der Weise eingestellt wird, daß die auf den Verstärkungsteil (5) aufgegebene Flüssigkeitsmenge nicht unter 30 % ihres Normalwertes sinkt.

6. Verfahren nach Anspruch einem der Ansprüche 1 bis 5, **dadurch gekennzeichnet, daß** die Mittelsiederfraktion (B) in flüssiger Form entnommen wird.

7. Verfahren nach einem der Ansprüche 1 bis 5, **dadurch gekennzeichnet, daß** der Brüdenstrom am unteren Ende der Trennwand (T) in der Weise eingestellt wird, daß das Verhältnis des Brüdenstroms im Zulaufteil (2, 4) zum Brüdenstrom im Entnahmeteil (3, 5) 0,8 bis 1,2, bevorzugt 0,9 bis 1,1, beträgt, vorzugsweise durch die Wahl und/oder Dimensionierung trennwirksamer Einbauten und/oder den Einbau druckverlusterzeugender Einrichtungen.

8. Verfahren nach einem der Ansprüche 1 bis 6, **dadurch gekennzeichnet, daß** der Rücklauf aus dem oberen gemeinsamen Kolonnenteil (1) in der Weise geregelt wird, daß das Verhältnis des Rücklaufstroms im Zulaufteil (2, 4) zum Rücklauf im Entnahmeteil (3,5) 0,1 bis 1, 0, bevorzugt 0,5 bis 0,8, beträgt.

9. Verfahren nach einem der Ansprüche 1 bis 8, **dadurch gekennzeichnet, daß** die Entnahme des Leichtsiederstroms (A) temperaturgeregelt erfolgt, wobei die Meßstelle für die Regeltemperatur im oberen gemeinsamen Teilbereich, (1) der Kolonne, an einer Stelle angeordnet ist, die um 3 bis 8, bevorzugt 4 bis 6 theoretische Trennstufen unterhalb des oberen Kolonnenendes angeordnet ist.

10. Verfahren nach einem der Ansprüche 1 bis 9, **dadurch gekennzeichnet, daß** die Enmahme des Hochsiederstroms (C) temperaturgeregelt erfolgt, wobei die Meßstelle für die Regeltemperatur im unteren gemeinsamen Kolonnenbereich (6), um 3 bis 8, bevorzugt um 4 bis 6 theoretische Trennstufen oberhalb des unteren Endes der Kolonne angeordnet ist.

11. Verfahren nach einem der Ansprüche 1 bis 10, **dadurch gekennzeichnet, daß** die Entnahme des Mittelsiederstroms (B) standgeregelt erfolgt und als Regelgröße der Flüssigkeitsstand im Verdampfer oder im Sumpf der Kolonne verwendet wird.

12. Verfahren nach einem der Ansprüche 1 bis 11, **dadurch gekennzeichnet, daß** die Flüssigkeitsverteilung in den einzelnen Teilbereichen 1 bis 6 der Trennwandkolonne (TK) jeweils getrennt einstellbar ist.

13. Verfahren nach einem der Ansprüche 1 bis 12, **dadurch gekennzeichnet, daß** in den Teilbereichen 2 und 4 der Trennwandkolonne (TK) Flüssigkeit verstärkt im Wandbereich und in den Teilbereichen 3 und 5 der Trennwandkolonne (TK) reduziert im Wandbereich aufgegeben wird.

14. Verwendung einer Trennwandkolonne (TK) mit 30 bis 100, bevorzugt 50 bis 90 theoretischen Trennstufen zur Durchführung des Verfahrens nach einem Ansprüche 1 bis 13.

15. Verwendung nach Anspruch 14, **dadurch gekennzeichnet, daß** die Kolonnenbereiche 1 bis 6 jeweils 5 bis 50 %, bevorzugt 15 bis 30 % der Gesamtzahl der theoretischen Trennstufen der Trennwandkolonne (TK) aufweisen.

16. Verwendung nach Anspruch 14 oder 15, **dadurch gekennzeichnet, daß** die Zulaufstelle des Stroms (A, B, C) und die Entnahmestelle des Mittelsiederstroms.(B) auf unterschiedlicher Höhe in der Kolonne angeordnet sind, vorzugsweise um 1 bis 20, insbesondere um 10 bis 15 theoretische Trennstufen beabstandet.

17. Verwendung nach einem der Ansprüche 14 bis 16, **dadurch gekennzeichnet, daß** einer oder mehrere der Teilbereiche 2, 3, 4 und 5 der Trennwandkolonne (TK) mit geordneten Packungen oder Füllkörpern bestückt ist (sind) und/oder, daß die Trennwand (T) in den an einen oder mehreren der Teilbereiche 2, 3, 4 und 5 angrenzenden Bereichen wärmeisolierend ausgeführt ist.

18. Steurwendung nach einem der Ansprüche 14 bis 17, **dadurch gekennzeichnet, daß** die Trennwand (T) in Form von lose gesteckten Teilsegmenten ausgestaltet ist.

19. Verwendung nach Anspruch 18, **dadurch gekennzeichnet, daß** die lose Trennwand (T) interne Mannlöcher oder herausnehmbare Segmente aufweist, die es erlauben, innerhalb der Trennwandkolonne (TK) von einer Seite der Trennwand (T) auf die andere Seite zu gelangen.

20. Verfahren nach Anspruch 1 unter Verwendung, von thermisch gekoppelten Kolonnen, **dadurch gekennzeichnet, daß** die beiden thermisch gekoppelten Kolonnen bei verschiedenen Drücken betrieben werden und/oder daß in den Verbindungsströmen zwischen den beiden thermisch gekoppelten Kolonnen nur Flüssigkeiten gefördert werden.

21. Verfahren nach Anspruch 20, **dadurch gekennzeichnet, daß** die Leichtsiederfraktion (A) und die Schwersiederfraktion (C) aus unterschiedlichen Kolonnen entnommen werden, und der Betriebsdruck der Kolonne, aus der die Hochsiederfraktion (C) entnommen wird, bevorzugt um 0,1 bis 2 bar, insbesondere um 0,5 bis 1 bar tiefer eingestellt wird als der Betriebsdruck der Kolonne, aus der die Leichtsiederfraktion (A) entnommen wird.

22. Verfahren nach einem der Ansprüche 1, 20 oder 21 unter Verwendung von thermisch gekoppelten Kolonnen, **dadurch gekennzeichnet, daß** der Sumpfstrom der ersten Kolonne in einem Verdampfer teilweise oder vollständig verdampft wird und anschließend der zweiten Kolonne zweiphasig oder in Form eines gasförmigen und eines flüssigen Stromes zugeführt wird.

23. Verwendung von thermisch gekoppelten Kolonnen mit jeweils eigenem Verdampfer und/oder Kondensator zur Durchführung des Verfahrens nach einem der Ansprüche 1 oder 20 bis 22.

24. Verwendung nach einem der Ansprüche 14 bis 19, **dadurch gekennzeichnet, daß** am oberen und am unteren Ende der Trennwand (TK) Probenahmemöglichl iten eingerichtet sind, über die aus der Kolonne kontinuierlich oder in zeitlichen Abständen flüssige und/oder gasförmige Proben entnommen werden und hinsichtlich ihrer Zusammensetzung, bevorzugt gaschromatographisch, untersucht werden.

## Claims

1. A process for working up by distillation the crude products obtained in the process according to DE-A 196 07 954 and comprising 1,6-hexanediol (HDO), 1,5-pentanediol (PDO) or caprolactone (CLO) in order to obtain the corresponding pure products, wherein the working-up by distillation is carried out in each case in a dividing wall column (TK) in which a dividing wall (T) is arranged in the longitudinal direction of the column with formation of an upper common column region (1), a lower common column region (6), a feed section (2, 4) having a rectification section (2) and stripping section (4), and a take-off section (3, 5) having a stripping section (3) and rectification section (5), with feeding of the respective crude product HDO, PDO or CLO in the middle region of the feed section (2, 4) and removal of a high boiler fraction (C) from the bottom of the column, of a low boiler fraction (A) via the top of the column and of a medium boiler fraction (B) from the middle region of the take-off section (3, 5), or in thermally coupled columns.

2. The process according to claim 1, wherein the distribution ratio of the liquid reflux at the upper end of the dividing wall (T) is set in a manner such that the proportion of high-boiling key components in the liquid reflux via the stripping section (3) of the take-off section (3, 5) at the upper end of the dividing wall (T) is from 10 to 80%, preferably from 30 to 50%, of the limit permitted in the medium boiler fraction (B), and wherein the heating power in the bottom evaporator of the dividing wall column (TK) is set in a manner such that the concentration of the low-boiling key components in the liquid at the lower end of the dividing wall (T) is from 10 to 80%, preferably from 30 to 50%, of the limit permitted in the medium boiler stream (B).

3. The process according to claim 1 or 2, wherein the HDO purification column and the PDO purification column are connected in a manner such that the HDO fractions remaining in the top stream of the HDO column are recovered via the bottom of the PDO purification column and are recycled to the HDO purification column.

4. The process according to any of claims 1 to 3, wherein the flow rate of the liquid which is fed to the middle region of the feed section (2, 4) is regulated in a manner such that it does not fall below 30% of its normal value.

5. The process according to any of claims 1 to 4, wherein the distribution of the liquid flowing out of the stripping section (3) of the take-off section (3, 5) of the dividing wall column (TK) between the medium boiler fraction (B) taken off and the rectification section (5) of the take-off section (3, 5) of the dividing wall column (TK) is established by regulation in a manner such that the amount of liquid added to the rectification section (5) does not fall below 30% of its normal value.

6. The process according to any of claims 1 to 5, wherein the medium boiler fraction (B) is taken off in liquid form.

7. The process according to any of claims 1 to 5, wherein the vapor stream at the lower end of the dividing wall (T) is set in a manner such that the ratio of the vapor stream in the feed section (2, 4) to the vapor stream in the take-off section (3, 5) is from 0.8 to 1.2, preferably from 0.9 to 1.1, preferably by the choice and/or dimensioning of internals having a separating effect and/or by the installation of means which generate a pressure drop.

8. The process according to any of claims 1 to 6, wherein the reflux from the upper common column section (1) is regulated so that the ratio of the reflux stream in the feed section (2, 4) to the reflux in the take-off section (3, 5) is from 0.1 to 1.0, preferably from 0.5 to 0.8.

9. The process according to any of claims 1 to 8, wherein the low boiler stream (A) is taken off in a temperature-controlled manner, the measuring point for the control temperature in the upper common region (1) of the column being arranged at a point which is from 3 to 8, preferably from 4 to 6, theoretical plates below the upper end of the column.

10. The process according to any of claims 1 to 9, wherein the high boiler stream (C) is taken off in a temperature-controlled manner, the measuring point for the control temperature in the lower common column region (6) being arranged from 3 to 8, preferably from 4 to 6, theoretical plates above the lower end of the column.

11. The process according to any of claims 1 to 10, wherein the medium boiler stream (B) is taken off under level control, and the liquid level in the evaporator or in the bottom of the column is used as a control variable.

12. The process according to any of claims 1 to 11, wherein the liquid distribution in the individual regions 1 to 6 of the dividing wall column (TK) can be set separately in each case.

13. The process according to any of claims 1 to 12, wherein more liquid is added in the wall region in the regions 2 to 4 of the dividing wall column (TK) and less liquid is added in the wall region in the regions 3 and 5 of the dividing wall column (TK).

14. The use of a dividing wall column (TK) comprising from 30 to 100, preferably from 50 to 90, theoretical plates for carrying out the process according to any of claims 1 to 13.

15. The use according to claim 14, wherein the column regions 1 to 6 each have from 5 to 50%, preferably from 15 to 30%, of the total number of theoretical plates of the dividing wall column (TK).

16. The use according to claim 14 or 15, wherein the feed point of the stream (A, B, C) and the take-off point of the medium boiler stream (B) are arranged at different heights in the column, preferably from 1 to 20, in particular from 10 to 15, theoretical plates apart.

17. The use according to any of claims 14 to 16, wherein one or more of the regions 2, 3, 4 and 5 of the dividing wall column (TK) is or are equipped with stacked packings or dumped packings and/or wherein the dividing wall (T) is made heat-insulating in the areas adjoining one or more of the regions 2, 3, 4 and 5.

18. The use according to any of claims 14 to 17, wherein the dividing wall (T) is in the form of loosely inserted segments.

19. The use according to claim 18, wherein the loose dividing wall (T) has internal manholes or removable segments which make it possible to reach from one side of the dividing wall (T) to the other side inside the dividing wall column (TK).

20. The process according to claim 1 using thermally coupled columns, wherein the two thermally coupled columns are operated at different pressures and/or wherein only liquids are transported in the connecting streams between the two thermally coupled columns.

21. The process according to claim 20, wherein the low boiler fraction (A) and the high boiler fraction (C) are taken off from different columns, and the operating pressure of the column from which the high boiler fraction (C) is taken off is set preferably from 0.1 to 2, in particular from 0.5 to 1, bar lower than the operating pressure of the column from which the low boiler fraction (A) is taken off.

22. The process according to any of claims 1, 20 and 21 using thermally coupled columns, wherein the bottom stream of the first column is partly or completely evaporated in an evaporator and is then fed to the second column in two-phase form or in the form of a gaseous and a liquid stream.

23. The use of thermally coupled columns each having their own evaporator and/or condenser for carrying out the process according to any of claims 1 and 20 to 22.

24. The use according to any of claims 14 to 19, wherein sampling facilities are set up at the upper and at the lower end of the dividing wall (TK), via which facilities liquid and/or gaseous samples are taken continuously or at time intervals from the column and are investigated with regard to their composition, preferably by gas chromatography.

## Revendications

1. Procédé de traitement par distillation de produits bruts contenant du 1,6-hexanediol (HDO), du 1,5-pentanediol (PDO) ou respectivement de la caprolactone (CLO), obtenus dans le procédé suivant la DE-A-196 07 954, pour la production des produits purs correspondants, **caractérisé en ce que** le traitement par distillation est effectué respectivement dans une colonne à paroi séparatrice (TK), dans laquelle une paroi séparatrice (T) est agencée dans la direction longitudinale de la colonne avec formation d'une zone de colonne commune supérieure (1), d'une zone de colonne commune inférieure (6), d'une partie d'introduction (2, 4) comportant une partie de renforcement (2) et une partie de rectification (4) ainsi que d'une partie de prélèvement (3, 5) comportant une partie de rectification (3) et une partie de renforcement (5), avec amenée du produit brut respectif HDO, PDO ou respectivement CLO dans la zone centrale de la partie d'introduction (2, 4), évacuation d'une fraction de substances à haut point d'ébullition (C) à partir du fond de colonne, d'une fraction de substances à bas point d'ébullition (A) par la tête de colonne et d'une fraction de substances à point d'ébullition moyen (B) à partir de la zone centrale de la partie de prélèvement (3, 5), ou dans des colonnes thermiquement couplées.

2. Procédé suivant la revendication 1, **caractérisé en ce que** le rapport de partage du reflux de liquide à l'extrémité supérieure de la paroi séparatrice (T) est ajusté de manière à ce que la fraction de composants clefs à point d'ébullition élevé dans le reflux de liquide au-dessus de la partie de rectification (3) de la partie de prélèvement (3, 5) soit à l'extrémité supérieure de la paroi séparatrice (T) de 10 à 80 %, de préférence de 30 à 50 %, de la valeur limite admise dans la fraction de substances à point d'ébullition moyen (B) et **en ce que** la puissance calorifique dans l'évaporateur de fond de la colonne à paroi séparatrice (TK) est ajustée de manière à ce que la concentration des composants clefs à bas point d'ébullition dans le liquide soit, à l'extrémité inférieure de la paroi séparatrice (T), de 10 à 80 %, de préférence de 30 à 50 %, de la valeur limite admise dans le courant de substances à point d'ébullition moyen (B).

3. Procédé suivant l'une des revendications 1 ou 2, **caractérisé en ce que** la colonne de HDO à l'état pur et la colonne de PDO à l'état pur sont montées de façon à ce que les fractions de HDO subsistant dans le courant de tête de la colonne de HDO soient obtenues au-dessus du fond de la colonne de PDO à l'état pur et soient recyclées dans la colonne de HDO à l'état pur.

4. Procédé suivant l'une des revendications 1 à 3, **caractérisé en ce que** le flux massique du liquide, qui est chargé dans la zone centrale de la partie d'introduction (2, 4), est réglé de manière à ce qu'il ne descende pas en dessous de 30 % de sa valeur normale.

5. Procédé suivant l'une des revendications 1 à 4,
**caractérisé en ce que** le partage du liquide sortant de la partie de rectification (3) de la partie de prélèvement (3, 5) de la colonne à paroi séparatrice (TK) en la fraction soutirée de substances à point d'ébullition moyen (B) et la partie de renforcement (5) de la partie de prélèvement (3, 5) de la colonne à paroi séparatrice (TK) est ajusté par un réglage de manière à ce que la quantité de liquide chargée à la partie de renforcement (5) ne descende pas en dessous de 30 % de sa valeur normale.

6. Procédé suivant l'une des revendications 1 à 5, **caractérisé en ce que** la fraction de substances à point d'ébullition moyen (B) est prélevée sous forme liquide.

7. Procédé suivant l'une des revendications 1 à 5, **caractérisé en ce que** le courant de buées à l'extrémité inférieure de la paroi séparatrice (T) est ajusté de manière à ce que le rapport entre le courant de buées dans la partie d'introduction (2, 4) et le courant de buées dans la partie de prélèvement (3, 5) soit de 0,8 à 1,2, de préférence de 0,9 à 1,1, de préférence par le choix et/ou le dimensionnement des éléments encastrés à action de séparation et/ou par l'incorporation de dispositifs produisant des pertes de pression.

8. Procédé suivant l'une des revendications 1 à 6, **caractérisé en ce que** le reflux provenant de la partie de colonne commune supérieure (1) est réglé de façon à ce que le rapport entre le courant de reflux dans la partie d'introduction (2, 4) et le reflux dans la partie de prélèvement (3, 5) soit de 0,1 à 1,0, de préférence de 0,5 à 0,8.

9. Procédé suivant l'une des revendications 1 à 8, **caractérisé en ce que** le prélèvement du courant de substances à bas point d'ébullition (A) a lieu d'une manière réglée en température, l'emplacement de mesure pour la température de réglage étant agencé dans la zone partielle commune supérieure (1) de la colonne, en un endroit qui est agencé à 3 jusqu'à 8, de préférence à 4 jusqu'à 6, étages séparateurs théoriques en dessous de l'extrémité supérieure de la colonne.

10. Procédé suivant l'une des revendications 1 à 9, **caractérisé en ce que** le prélèvement du courant de substances à haut point d'ébullition (C) a lieu d'une manière réglée en température, l'emplacement de mesure pour la température de réglage étant agencé dans la zone de colonne commune inférieure (6) vers 3 à 8, de préférence 4 à 6, étages séparateurs théoriques au-dessus de l'extrémité inférieure de la colonne.

11. Procédé suivant l'une des revendications 1 à 10, **caractérisé en ce que** le prélèvement du courant de substances à point d'ébullition moyen (B) a lieu d'une manière réglée en niveau et on utilise, comme grandeur de réglage, le niveau de liquide dans l'évaporateur ou dans le fond de la colonne.

12. Procédé suivant l'une des revendications 1 à 11, **caractérisé en ce que** la répartition du liquide dans les différentes zones partielles 1 à 6 de la colonne à paroi séparatrice (TK) peut chaque fois être ajustée de manière séparée.

13. Procédé suivant l'une des revendications 1 à 12, **caractérisé en ce que**, dans les zones partielles 2 et 4 de la colonne à paroi séparatrice (TK), du liquide est chargé de manière renforcée dans la zone de paroi et, dans les zones partielles 3 et 5 de la colonne à paroi séparatrice (TK), il est chargé de manière réduite dans la zone de paroi.

14. Utilisation d'une colonne à paroi séparatrice (TK) comportant 30 à 100, de préférence 50 à 90, étages séparateurs théoriques, pour la mise en oeuvre du procédé suivant l'une des revendications 1 à 13.

15. Utilisation suivant la revendication 14, **caractérisée en ce que** les zones 1 à 6 de la colonne présentent chacune 5 à 50 %, de préférence 15 à 30 %, du nombre total des étages séparateurs théoriques de la colonne à paroi séparatrice (TK).

16. Utilisation suivant la revendication 14 ou 15, **caractérisée en ce que** l'emplacement d'introduction du courant (A, B, C) et l'emplacement de prélèvement du courant de substances à point d'ébullition moyen (B) sont agencés à des hauteurs différentes dans la colonne, de préférence avec un écartement de 1 à 20, en particulier de 10 à 15, étages séparateurs théoriques.

17. Utilisation suivant l'une des revendications 14 à 16, **caractérisée en ce qu'**une ou plusieurs des zones partielles 2, 3, 4 et 5 de la colonne à paroi séparatrice (TK) sont équipées de corps de remplissage ou de garnissages ordonnés et/ou **en ce que** la paroi séparatrice (T) est réalisée sous une forme thermiquement isolante dans les zones adjacentes à une ou plusieurs des zones partielles 2, 3, 4 et 5.

18. Utilisation suivant l'une des revendications 14 à 17, **caractérisée en ce que** la paroi séparatrice (T) est conformée sous la forme de segments partiels incorporés de manière amovible.

19. Utilisation suivant la revendication 18, **caractérisée en ce que** la paroi séparatrice (T) amovible présente des trous d'homme internes ou des segments détachables qui permettent de parvenir à l'intérieur de la colonne à paroi séparatrice (TK) d'un côté de la paroi séparatrice (T) à l'autre côté.

20. Procédé suivant la revendication 1, avec utilisation de colonnes thermiquement couplées, **caractérisé en ce que** les deux colonnes thermiquement couplées sont mises en service à des pressions différentes et/ou **en ce que** seuls des liquides sont transportés dans les courants qui communiquent entre les deux colonnes thermiquement couplées.

21. Procédé suivant la revendication 20, **caractérisé en ce que** la fraction de substances à bas point d'ébullition (A) et la fraction à substances à haut point d'ébullition (C) sont prélevées à partir de colonnes différentes et la pression de service de la colonne à partir de laquelle la fraction de substances à haut point d'ébullition (C) est prélevée est ajustée de préférence à 0,1 jusqu'à 2 bars, en particulier de 0,5 jusqu'à 1 bar, en dessous de la pression de service de la colonne à partir de laquelle la fraction de substances à bas point d'ébullition (A) est prélevée.

22. Procédé suivant l'une des revendications 1, 20 et 21, avec utilisation de colonnes thermiquement couplées, **caractérisé en ce que** le courant de fond de la première colonne est évaporé partiellement ou totalement dans un évaporateur et ensuite est amené à la deuxième colonne à un état biphasique ou sous la forme d'un courant gazeux et d'un courant liquide.

23. Utilisation de colonnes thermiquement couplées avec chacune un évaporateur et/ou condenseur propre, pour la mise en oeuvre du procédé suivant l'une des revendications 1 ou 20 à 22.

24. Utilisation suivant l'une des revendications 14 à 19, **caractérisée en ce que**, à l'extrémité supérieure et à l'extrémité inférieure de la paroi séparatrice (TK), sont disposées des possibilités de prise d'échantillons par lesquelles des échantillons liquides et/ou gazeux sont prélevés de la colonne de manière continue ou à intervalles de temps et sont examinés quant à leur composition, de préférence par chromatographie en phase gazeuse.
